## Europäisches Patentamt
## European Patent Office
## Office européen des brevets

(19)

(11) Veröffentlichungsnummer : **0 116 568
B1**

(12) **EUROPÄISCHE PATENTSCHRIFT**

(45) Veröffentlichungstag der Patentschrift :
05.04.89

(51) Int. Cl.⁴ : **A 61 M  5/14**

(21) Anmeldenummer : **83902252.2**

(22) Anmeldetag : **21.07.83**

(86) Internationale Anmeldenummer :
**PCT/EP 83/00192**

(87) Internationale Veröffentlichungsnummer :
**WO/8400493 (16.02.84 Gazette 84/05)**

(54) **GERÄT ZUR GEREGELTEN INFUSION VON FLÜSSIGKEITEN.**

(30) Priorität : **23.07.82 DE 3227518**

(43) Veröffentlichungstag der Anmeldung :
**29.08.84 Patentblatt 84/35**

(45) Bekanntmachung des Hinweises auf die Patenterteilung : **05.04.89 Patentblatt 89/14**

(84) Benannte Vertragsstaaten :
**AT BE CH DE FR GB LI LU NL SE**

(56) Entgegenhaltungen :
**EP--A-- 0 002 776
EP--A-- 0 028 929
US--A-- 4 146 029
Electronique Applications, Nr. 14, 1980, Soc. Parisienne d'éditions, Paris (FR) J. Tremolières: "Ordinateur pour analgésie sur demande"; Seiten 53-56**

(73) Patentinhaber : **STOFFREGEN, Jürgen
Am Teich 11
D-5800 Hagen-Hassley (DE)**

(72) Erfinder : **STOFFREGEN, Jürgen
Am Teich 11
D-5800 Hagen-Hassley (DE)**

(74) Vertreter : **König, Norbert, Dipl.-Phys. Dr. et al
Patentanwälte Leine & König Burckhardtstrasse 1
D-3000 Hannover 1 (DE)**

EP 0 116 568 B1

**Beschreibung**

Die Erfindung betrifft ein Gerät zur geregelten Infusion von Flüssigkeiten gemäß Oberbegriff des Anspruchs 1.

Durch die Zeitschrift « Pharmaceutical Journal », Okt. 1978, ist ein unter dem Namen « on demand analgesia computer » eingeführtes Verfahren bekannt geworden, bei dem dem Patienten ein Analgetikum stetig infundiert wird und bei dem der Patient die Möglichkeit hat, selbst durch Betätigung eines Bedienungsknopfes zusätzliche Analgetikumdosen zu infundieren. Gesteuert wird das Verfahren über einen entsprechend programmierten Computer, wobei der Patient in regelmäßigen Zeitabständen über ein laufendes Tonband befragt wird und seine Antwort durch Drücken eines Knopfes an den Rechner weitergeben kann, der daraus den Bedarf an Analgetikum ermittelt. Für eine vorgegebene Zeit wird dabei die infundierte Dosis mit einer für diese Zeit vorgegebenen Maximaldosis verglichen und die Infusion gegebenenfalls automatisch gestoppt. Bei diesem bekannten Verfahren ist nachteilig, daß der Bedarf wesentlich durch die verbale Befragung des Patienten ermittelt wird. Damit wird das Verfahren natürlicherweise recht ungenau, und es sind keine reproduzierbaren Ergebnisse zu erwarten. Ein entscheidender Nachteil ist dabei, daß über einen längeren Zeitraum die Gefahr einer Überdosierung besteht, die nur durch periodisches Nachregeln per Hand vermeidbar ist.

Durch die EP-A 0 002 776 ist eine Vorrichtung zur vorprogrammierbaren Infusion von Flüssigkeiten für die Diabetes-Therapie bekannt. Diese bekannte Vorrichtung weist eine Steuervorrichtung auf, die als Programmgeber für eine Mikrodosiereinheit dient. Bei einem vorgegebenen Programm ist der Patient gezwungen, die Einnahmen von Mahlzeiten und dgl. nach dem vorprogrammierten Steuerprogramm einzuhalten. Der Steuervorrichtung für die Mikrodosiereinheit sind Speichermittel für ein vorgebbares Steuerprogramm zugeordnet, wobei das Steuerprogramm an der Steuervorrichtung vom Patienten direkt abrufbar ist. Dabei wird lediglich durch Anwahl einer Anzahl der in den Körper abzugebenden Insulineinheiten bzw. der vom Patienten mit einer Mahlzeit aufgenommenen Broteinheiten und eines Startzeitpunktes der programmäßige Ablauf der Infusion gestartet. Zusätzlich können vom Patienten bestimmte Basisraten der Infusion angewählt werden. Mit dieser bekannten Vorrichtung können Diabetes-Patienten bei Mahlzeiten notwendige Insulinverabreichungen nach kurzen Programmen abrufen. Das Steuerprogramm der Infusionsrate entspricht im wesentlichen einer Dreiecksform, beispielsweise rechtwinklig, wobei die Infusionsrate von einem Basiswert schnell auf den Höchstwert und anschließend vom Höchstwert mit vorgegebener funktionaler Abhängigkeit wieder auf den Basiswert abnimmt. Der Abnahmegrad ist dabei vorzugsweise linear mit vorgegebener Steigung. Er kann aber auch exponential nach Art einer e-Funktion sein.

Die Aufgabe der vorliegenden Erfindung besteht darin, das Gerät gemäß Oberbegriff des Anspruchs 1 so auszubilden, daß die Infusionsrate über einen längeren Zeitraum automatisch gesteuert und geregelt werden kann, die Gefahr einer Überdosierung vermieden und genauere und reproduzierbare Ergebnisse erzielbar sind ; außerdem soll eine manuelle Eingriffsmöglichkeit geschaffen werden zur definierten Änderung der Infusionsrate.

Diese Aufgabe wird durch die im Anspruch 1 angegebene Ausbildung gelöst.

Durch die vorliegende Erfindung läuft der Infusionsvorgang praktisch automatisch ab. Es werden genaue, reproduzierbare Infusionsergebnisse erzielt. Die Gefahr einer Überdosierung ist sicher vermieden, da über eine vorbestimmbare Zeit eine zwangsläufig per Programm einsetzende Verringerung der Dosis auf einen vorgebbaren geringeren oder minimalen Dosiswert vorgesehen ist. Das erfindungsgemäße Gerät ist so konzipiert, daß auch eine manuelle Einflußnahme auf den Infusionsvorgang möglich ist, dergestalt, daß je nach Bedarf in genau definierter, vorbestimmter Weise zusätzliche Infusionsimpulse ausgelöst werden können. Durch die Erfindung kann jedes gewünschte Infusionsprofil realisiert werden. Da Eingriffsmöglichkeiten auch in das laufende Programm vorgesehen sind, ist es möglich, auch das vorgesehene Infusionsprofil laufend an die gegebenen Bedingungen anzupassen.

Weiterbildungen der Erfindung sind in den Unteransprüchen gekennzeichnet.

Die Erfindung soll nachfolgend anhand der beigefügten Zeichnung näher erläutert werden.

Es zeigt

Fig. 1 schematisch eine Ansicht des erfindungsgemäßen Gerätes,

Fig. 2 ein Blockschaltbild des Gerätes nach Fig. 1,

Fig. 3, 4 und 5 grafische Darstellungen zur Erläuterung der Wirkungsweise des Gerätes in verschiedenen Betriebsarten,

Fig. 6 ein Blockschaltbild einer anderen Ausführungsform des erfindungsgemäßen Gerätes.

Gleiche Teile sind in den Figuren der Zeichnung mit den gleichen Bezugszeichen versehen.

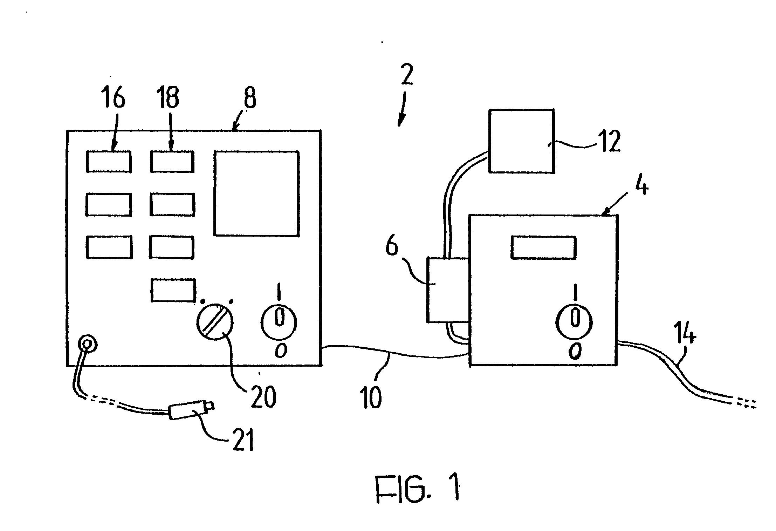

Die Fig. 1 zeigt ein Infusionsgerät 2, das im wesentlichen aus einer Infusionspumpe 4 mit Tropfen- oder Durchflußmengenzähleinrichtung 6 und einem Programm- und Steuersignalgeber 8 besteht, über den die Infusionspumpe über eine elektrische Steuerleitung 10 nach einem austauschbaren oder veränderbaren Programm angetrieben wird. Die Infusionspumpe ist an einen Flüssigkeitsvorratsbehälter 12 angeschlossen und fördert die Flüssigkeit in eine Infusionsleitung 14.

Auf der Frontplatte des Gerätes sind verschiedene Drucktasten 16 zum Voreinstellen verschiedener Parameter für das gewünschte Infusionsprofil angeordnet sowie verschiedene Zähleranzeigen 18 zur Kontrolle des Betriebs des Gerätes. Ein Programmschalter 20 dient zur Umschaltung zwischen einem vollautomatischen Betrieb und einem Betrieb nach Bedarf, bei dem beispielsweise die Möglichkeit besteht, über eine Drucktaste 21 die Infusionsrate für eine vorgebbare Zeit zu ändern, d. h. also zusätzliche Infusionsimpulse auszulösen.

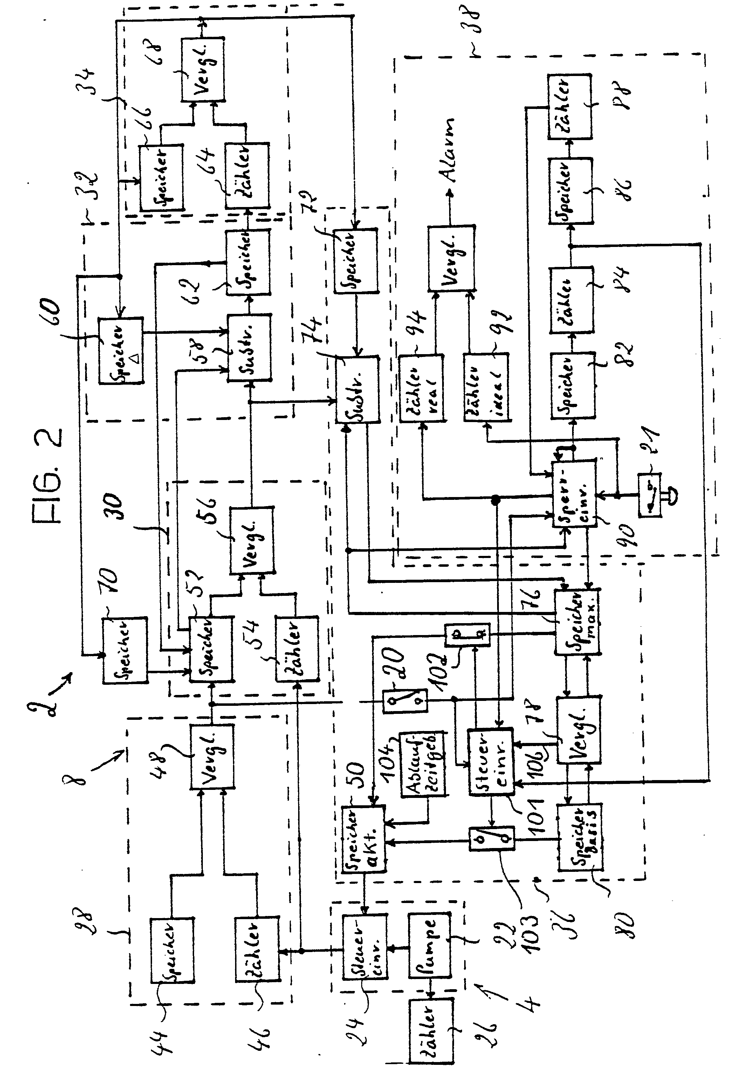

Die Fig. 2 zeigt das Infusionsgerät 2 im Blockschaltbild in seinen wesentlichen Bestandteilen. Die Infusionspumpe 4 besteht aus der eigentlichen Pumpe 22, einer Steuer- und Überwachungseinrichtung 24 sowie einem Tropfen- oder Durchflußmengenzähler 26.

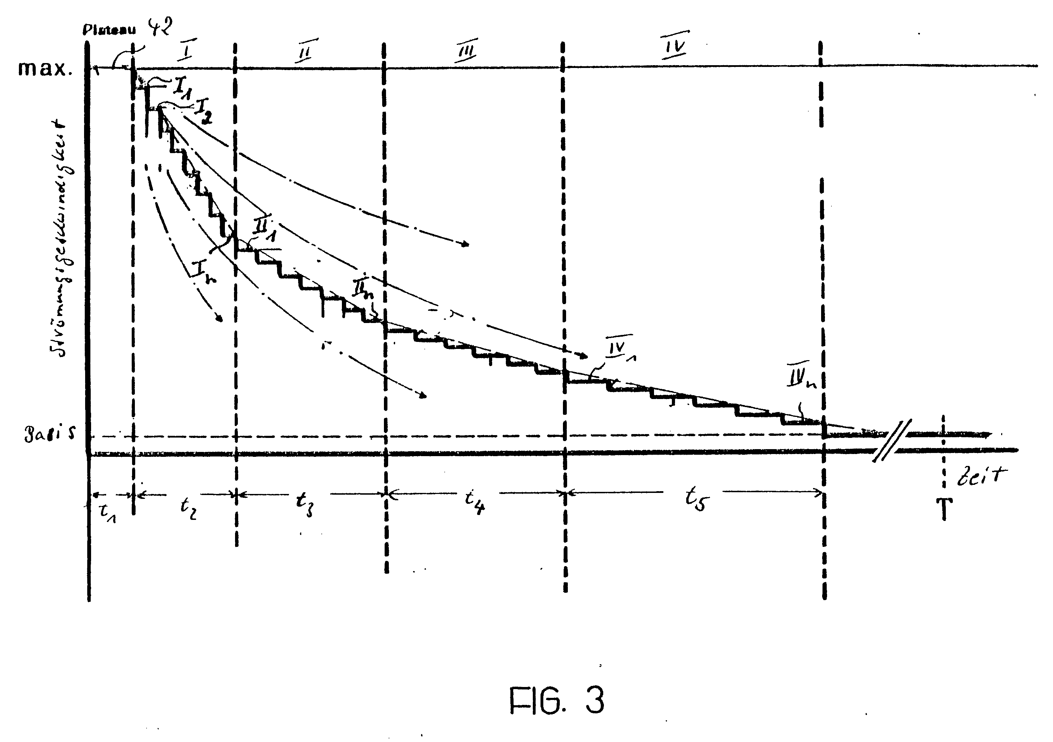

Der Programm- und Steuersignalgeber 8 besteht aus verschiedenen Funktionseinheiten 28, 30, 32, 34, 36 und 38, die in vorbestimmbarer Weise zusammenarbeiten, um ein bestimmtes Infusionsprofil 40 zu erhalten (Fig. 3 bis 5).

Die Funktionseinheit 28 liefert den ersten Teil des Infusionsprofiles, das sogenannte Plateau 42 mit maximaler Infusionsrate (vgl. Fig. 3 bis 5). Hierzu ist ein auf die gewünschte Flüssigkeitsmenge voreinstellbarer Speicher 44 vorgesehen, dessen Inhalt mit der laufenden in einem Zähler 46 festgehaltenen infundierten Menge in einem Soll-/Istwertvergleicher 48 verglichen wird. Die jeweils gewünschte maximale Infusionsrate (Tropfen oder Milliliter pro Zeiteinheit) wird in einen Speicher 76 eingegeben, dessen Inhalt über einen normalerweise geschlossenen Schalter 102 in einen Speicher 50 geladen wird, der zur Abspeicherung des jeweils aktuellen Strömungsgeschwindigkeitswertes für die Infusionspumpe 22 dient und dessen Ausgangssignal über die Steuer- und Überwachungseinrichtung 24 die Pumpe 22 entsprechend steuert. Sobald der Istwert den Sollwert erreicht hat, gibt der Vergleicher 48 ein Steuersignal an die Funktionseinheit 30 ab.

Die Funktionseinheit 30 bestimmt zusammen mit den Funktionseinheiten 32 und 34 das Infusionsprofil in den sich an das Plateau 42 anschließenden Infusionsintervallen I, II, III, IV (vgl. Fig. 3 bis 5). Das Steuersignal des Vergleichers 48 aktiviert die Funktionseinheit 30. Der Inhalt eines beispielsweise die jeweiligen Flüssigkeitsmengenwerte aufeinanderfolgender Intervallstufen $I_1$-$I_n$, ..., $IV_1$-$IV_n$ (vgl. Fig. 3 bis 4) enthaltenden Speichers 52 wird mit der laufenden Istmenge, die von einem Zähler 54 ermittelt wird, in einem Vergleicher 56 verglichen. Sobald der Istwert den Sollwert erreicht, wird ein Steuersignal an einen Subtrahierer 58 der Funktionseinheit 32 gegeben, welcher vom Inhalt des Speichers 52 einen bestimmten Wert abzieht, der in einem Speicher 60 für Mengenreduzierung abgespeichert ist. Der verringerte Mengenwert wird in einen Zwischenspeicher 62 gegeben und von dort in den Speicher 52 eingeschrieben. Er stellt den Sollwert für die nächste Stufe des Intervalles, beispielsweise der Stufe $I_2$ des Intervalles I (vgl. Fig. 3) dar. Es erfolgt wieder der beschriebene Soll-/Istwertvergleich mit nachfolgender Verringerung des Mengenvorgabewertes für den Speicher 52, bis eine vorbestimmte Stufenzahl erreicht ist. Diese wird in der Funktionseinheit 34 ermittelt, die einen Zähler 64 aufweist, der die Zahl der Subtraktionsvorgänge zählt und dessen Zählerstand laufend mit der in einem Speicher 66 abgespeicherten, dem jeweiligen Intervall entsprechenden vorbestimmten Soll-Stufenzahl in einem Vergleicher 68 verglichen wird. Wird die vorbestimmte Stufenzahl, beispielsweise n erreicht, steuert der Vergleicher 68 Speicher 70, 72 und die Speicher 60 und 66 an.

Der Speicher 70, der Teil der Funktionseinheit 36 ist, enthält die Mengenvorgabewerte für die einzelnen Intervalle, beispielsweise I-IV, die aufeinanderfolgend nach Durchlaufen jedes Intervalles in den Speicher 52 gegeben werden. Der Speicher 60 wird auf einen dem jeweils nächsten Intervall zugeordneten Verringerungswert gesetzt. Der Speicher 72 enthält Werte (ggf. unterschiedliche), um die die im Speicher 76 abgespeicherten jeweils höchsten Infusionsraten von Intervall zu Intervall erniedrigt werden sollen. Hierzu ist ein Subtrahierer 74 vorgesehen, dessen Ausgangsgröße, die der Differenz der Inhalte der Speicher 76 und 72 entspricht, wieder in den Speicher 76 gegeben wird und in einem Vergleicher 78 mit einem minimalen Basis-Strömungsgeschwindigkeitswert verglichen wird, der in einem Basiswertspeicher 80 abgespeichert ist.

Ist der im Subtrahierer 74 erhaltene Infusionsratenwert bzw. Strömungsgeschwindigkeitswert größer als der Basiswert, wird der Infusionsratenwert aus dem Speicher 76 in den Speicher 50 als der aktuelle Wert geladen, und zwar über den geschlossenen Schalter 102. Ist er gleich oder kleiner als der Basiswert, wird letzterer in den Speicher 50 geladen, und zwar über einen normalerweise offenen, zwischen den Speichern 80 und 50 angeordneten Schalter 103, der durch eine Steuereinrichtung 101 umgeschaltet, d. h. geschlossen wird, welche gleichzeitig auch den normalerweise geschlossenen Schalter 102 umschaltet, d. h. öffnet. Die Steuereinrichtung 101 wird vom Vergleicherausgangssignal über eine Leitung 106 betätigt. Der jeweils im Speicher 50 stehende Wert bestimmt die jeweilige Infusionsrate der Infusionspumpe bzw. die jeweilige Strömungsgeschwindigkeit der von der Infusionspumpe infundierten Flüssigkeit, wie oben schon erläutert worden ist.

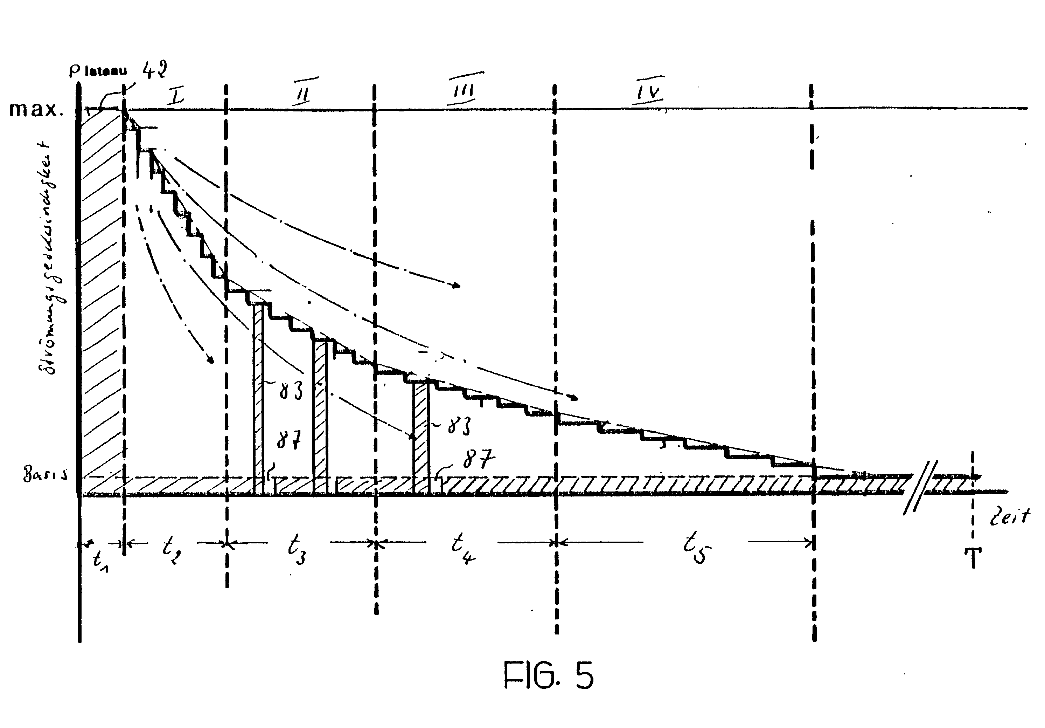

Nach Erreichen des konstanten Basis-Geschwindigkeitswertes und nach Ablauf einer vorbestimmbaren Zeit T, auf die ein Ablauf-Zeitgeber 104 einstellbar ist (vgl. auch Fig. 3 und 5), wird das Infusionsgerät 2 automatisch abgeschaltet.

Da die maximale Infusionsrate bzw. Strömungsgeschwindigkeit, die Flüssigkeitsmengenvorgabe für das Plateau und die einzelnen Intervalle und Intervallstufen sowie die Stufenzahl pro Intervall praktisch beliebig vorgebbar sind, kann auch jedes gewünschte Infusionsprofil realisiert werden, vgl. hierzu Fig. 3, in der beispielsweise die einzelnen Intervalle unterschiedlich lang und die Intervallstufen von Intervall zu Intervall in der Höhe abnehmen.

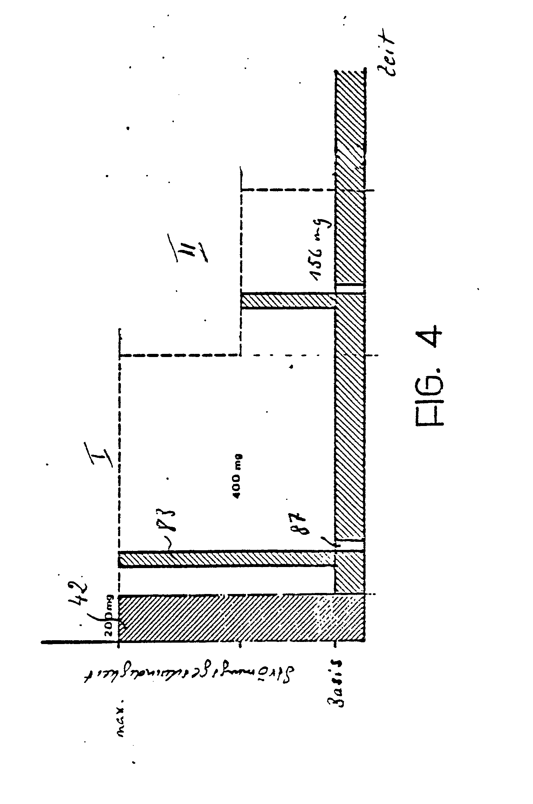

Das Infusionsgerät 2 sieht neben der automatischen Betriebsweise, die vorgehend beschrieben worden ist, noch eine sogenannte Infusion nach Bedarf vor, bei der die Möglichkeit besteht, unter bestimmten Voraussetzungen und in bestimmten Grenzen die Infusionsrate zu beeinflussen. Hierzu sind zusätzlich im wesentlichen die Funktionseinheit 38 und weitere Bauteile der Schaltungsanordnung 36 vorgesehen. Mit Hilfe des Programmschalters 20 wird zunächst von der Automatikbetriebsart auf die Bedarfsbetriebsart umgeschaltet, d. h. der Schalter 20 wird geschlossen. Der Bedarfsbetrieb ist aber erst möglich nach dem Setzen, d. h. Freigeben einer Sperreinrichtung 90, die normalerweise gesperrt ist. Dies erfolgt durch ein Ausgangssignal des Vergleichers 48, also nach Durchlaufen des Plateaus 42, vgl. Fig. 4 und 5. Das Ausgangssignal des Vergleichers 48 wird, da der Schalter 20 geschlossen ist, außerdem der Steuereinrichtung 101 zugeführt, die zur Steuerung des normalerweise geschlossenen Schalters 102, der zwischen den Speichern 76 und 50 angeordnet ist, und des zwischen den Speichern 80 und 50 angeordneten, im Bedarfsbetrieb normalerweise offenen Schalters 103 vorgesehen ist. Durch das Ausgangssignal des Vergleichers 48 wird über die Steuereinrichtung 101 eine Umschaltung des Schalters 102 in den geöffneten Zustand und des Schalters 103 in den geschlossenen Zustand bewirkt, wodurch von maximaler Infusionsrate (Plateau) auf die Basisinfusionsrate umgeschaltet wird. Über den Druckschalter 21 kann nach Freigabe der Sperreinrichtung 90 ein Speicher 82 angesteuert werden, in dem veränderbare, abrufbare Werte gespeichert sind, die vorgegebenen Flüssigkeitsmengen für die einzelnen Intervalle entsprechen, die bei Bedarf per Knopfdruck in einer vorgegebenen Zeit zusätzlich infundiert werden können, wobei die Infusionsrate gleich der aktuellen, dem jeweiligen Infusionsintervall oder der jeweiligen Infusionsintervallstufe zugeordneten Rate ist, die laufend über das oben beschriebene Automatikprogramm ermittelt wird oder gesondert per Programm bestimmt wird, vgl. hierzu die Säulen 83 in den Fig. 4 und 5.

Sobald ein (realer) Druckschalter-Bedarfsimpuls die Sperreinrichtung passiert, wird gleichzeitig mit dem Ansteuern des Speichers 82 die Steuereinrichtung 101 angesteuert, die eine erneute Umsteuerung der Schalter 102 und 103 bewirkt ; d. h. Schalter 102 schließt und Schalter 103 öffnet.

Mit dem Setzen des Speichers 82 läuft ein Zeitglied oder ein Zähler 84 ab, dessen Zeitkonstante oder Zählerstand dem jeweiligen aktuellen Inhalt des Speichers 82 zugeordnet ist. Nach Ablauf des Zeitgliedes oder nach Erreichen des Zählerstandes Null des Zählers wird wiederum die Steuereinrichtung 101 betätigt, die den zwischen Speicher 80 und Speicher 50 angeordneten Schalter 103 und gleichzeitig den Schalter 102 erneut umschaltet, d. h. der Schalter 103 wird geschlossen und Schalter 102 geöffnet, so daß über den Speicher 80 wieder der Basiswert für die Strömungsgeschwindigkeit in den Speicher 50 geladen und die Pumpe 22 entsprechend über die Steuer- und Überwachungseinrichtung 24 gesteuert wird. Gleichzeitig wird ein weiterer Speicher 86 gesetzt, in den veränderbare Werte abrufbar gespeichert sind, die einer Flüssigkeitsmenge für eine nachfolgende sogenannte refraktäre Pause 87 entsprechen, in der mit der Basisinfusionsrate infundiert wird. Mit Ansteuerung des Speichers 86 wird ein auf die refraktäre Pausenzeit einstellbares Zeitglied oder einstellbarer Zähler 88 angesteuert, das bzw. der abläuft und nach Ablauf bzw. Erreichen des Zählerstandes Null ein Schaltsignal abgibt, das der Sperreinrichtung 90 zugeführt wird.

Nach Ansteuerung des Speichers 82 und der Steuereinrichtung 101 wird die Sperreinrichtung 90 wieder in den Sperrzustand geschaltet, damit während der erhöhten Infusion nicht ein weiterer Infusionsvorgang mit erhöhter Infusionsrate gestartet werden kann. Erst nach Ablauf der refraktären Pause 87, d. h. nach Ablauf des Zählers 88, wird die Sperreinrichtung durch das Ausgangssignal dieses Zählers wieder in den Durchlaßbetrieb geschaltet.

Ein wirksames Bedarfssignal des Druckschalters 21 steuert gleichzeitig den Speicher 76 an und bewirkt die Übertragung des jeweiligen maximalen Strömungsgeschwindigkeitswertes in den Speicher 50 zur entsprechenden Steuerung der Infusionspumpe 22.

Jeder Tastendruck des Schalters 21 wird in einem Zähler 92 und jedes wirksame, den Speicher 82 ansteuernde Bedarfssignal in einem Zähler 94 gezählt. Die Differenz beider Zählerstände wird laufend in einem Vergleicher 96 ermittelt. Das der Differenz entsprechende Ausgangssignal ist ein Maß für ein bestimmtes Defizit. Wenn dieses Defizit einen bestimmten Betrag übersteigt, wird ein Alarmsignal ausgelöst, welches dazu verwendet wird, die refraktären Pausenwerte im Speicher 86 und Zähler 88 manuell oder automatisch zu korrigieren, d. h. zu verkleinern, um so die Sperreinrichtung 90 wieder schneller in den Durchlaßzustand zu schalten und um damit die praktisch realisierbare Häufigkeit der Bedarfsinfusionsimpulse zu erhöhen.

Nach Ablauf des Bedarfsbetriebs wird der im Speicher 80 abgespeicherte Basis-Strömungsgeschwindigkeitswert über den Vergleicher 78 in den Speicher 76 für den jeweils höchsten Strömungsgeschwindigkeitswert abgespeichert, so daß der Speicher 50 auch bei Freigabe der Sperreinrichtung 90 im Bedarfsfalle nur den Basis-Strömungsgeschwindigkeitswert erhält und die Infusionspumpe 22 dementsprechend auch nur mit der Basisinfusionsrate arbeitet.

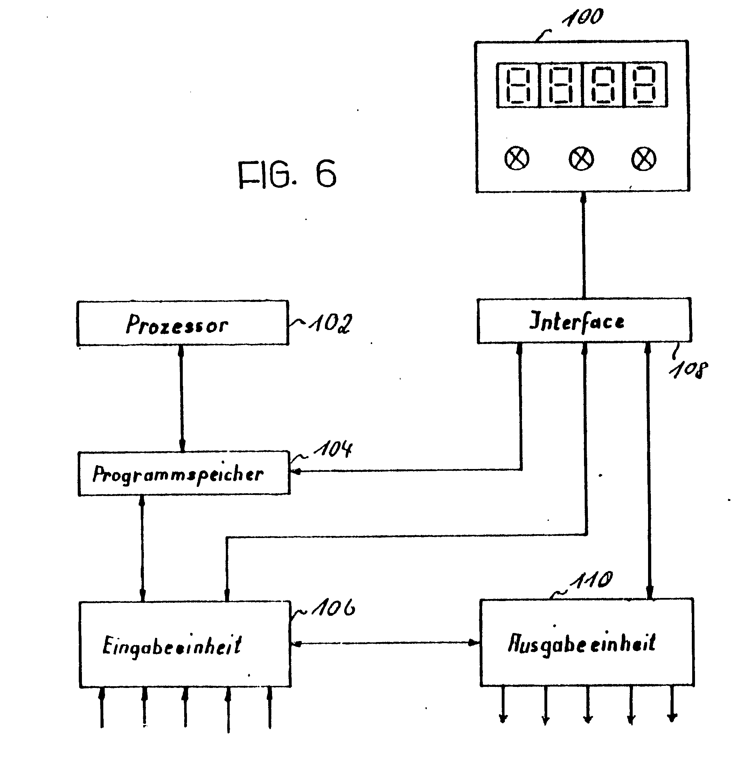

Die Fig. 6 zeigt eine Anordnung, bei der eine Infusionsmaschine 100 über ein vorgegebenes Programm von einem Prozessor 102 gesteuert wird, der sämtliche Steuersignale erzeugt, die zur Steuerung der Infusionsmaschine nach einem vorgegebenen Infusionsprofil notwendig sind. Das Infusionsprofil und das notwendige Programm sind in einem Speicher 104 abgespeichert, wobei über eine Eingabeeinheit 106 eine Änderung des Programmes und des Infusionsprofiles möglich ist. Ein Interface 108 stellt die Verbindung zwischen Infusionsmaschine und Prozessor sowie zu einer Ausgabeeinheit 110 her, mit der sämtliche angegebenen Daten, die laufenden Werte während des Infusionsvorganges und das Infusionsprofil wiedergebbar sind.

Sämtliche eingesetzten Speicher sind vorzugsweise Schreib-/Lesespeicher. Die Infusion der Flüssigkeit und die Messung der infundierten Menge erfolgt in Tropfen pro Zeiteinheit, beispielsweise Tropfen/min. oder in Milliliter pro Zeiteinheit, beispielsweise ml/h.

Es soll nochmals Bezug genommen werden auf die Fig. 3, 4 und 5. Die Fig. 3 zeigt ein vollständiges Infusionsprofil für einen vollautomatischen Infusionsbetrieb ohne Eingriffsmöglichkeit über den Bedarfsschalter 21. Es besteht im Prinzip aus dem bereits erwähnten Plateau 42 mit maximaler Infusionsgeschwindigkeit über eine vorbestimmte Zeit $t_1$. An dieses Plateau schließen sich je nach Aufgabe mehrere Infusionsintervalle I bis IV mit vorbestimmten Zeiten $t_2$ bis $t_5$ an, in denen die infundierte Flüssigkeitsmenge stufenweise verringert wird, was beispielsweise durch stufenweise Verringerung der Infusionsrate bzw. Strömungsgeschwindigkeit für gleiche Stufenzeiten pro Intervall erreicht wird. An das letzte Intervall IV schließt sich eine Phase konstanter, minimaler Infusionsgeschwindigkeit (Basiswert) an. Nach Ablauf einer vorbestimmten Gesamtzeit T wird die Infusion beendet.

Die Fig. 4 zeigt einen Teil eines Infusionsprofils, das im Bedarfsbetrieb, also mit sogenannter Patientenrückkopplung, d. h. durch manuellen Eingriff, d. h. durch Betätigung des Schalters 21 (Fig. 2) erhalten werden kann. Nach Durchfahren des üblicherweise vorgesehenen Plateaus 42 wird die Infusionsgeschwindigkeit auf den Basiswert abgesenkt. Es besteht dann die Möglichkeit, nach Bedarf in den einzelnen Intervallen über eine vorbestimmte Zeit mit der für das betreffende Intervall vorgesehenen höchsten Infusionsgeschwindigkeit eine vorgegebene Menge an Flüssigkeit (Säule 83) zusätzlich zu infundieren. Nach jeder solcher zusätzlichen kurzzeitigen Bedarfsinfusion (Infusionsimpuls) ist eine sogenannte refraktäre Pause 87 vorgesehen, in der die Infusionsgeschwindigkeit auf den Basiswert (oder auch Null) abgesenkt wird und in der keine weitere Bedarfsinfusion, d. h. kein weiterer Infusionsimpuls möglich ist. Erst nach Ablauf dieser refraktären Pause 87 ist wieder eine Bedarfsinfusion möglich. Im zweiten Intervall sind maximale Infusionsgeschwindigkeit bzw. Tropf- oder Strömungsgeschwindigkeit pro Bedarfsinfusion und Gesamtmenge verkleinert. Gemäß Fig. 4 erfolgt nach dem Intervall II eine Absenkung auf die Basisinfusionsgeschwindigkeit, so daß hier ein zusätzlicher Infusionsimpuls mit höherer Infusionsrate nich mehr möglich ist.

Die Fig. 5 zeigt ein Infusionsprofil, das im Prinzip dem nach Fig. 4 ähnlich ist ; nur ist hier nicht nur eine Verringerung der Infusionsgeschwindigkeit und der Gesamtinfusionsmenge von Intervall zu Intervall vorgesehen, sondern in jedem Intervall wird diese Größe bereits im Prinzip wie beim Automatikprogramm nach Fig. 3 stufenweise verringert, so daß praktisch hinsichtlich der maximalen Infusionsgeschwindigkeit auch noch für jedes Intervall eine mehrfache Abstufung vorgesehen ist. Diese Abstufung kann sich, wie dargestellt, von Intervall zu Intervall ändern, um beispielsweise eine e-Funktion nachzubilden.

Das oben beschriebene Infusionsgerät kann wie folgt eingesetzt werden :

a) Intraoperativ : Kombinationsnarkose mit kontrollierter Schmerzmittel-Infusion mit Verringerung ;

b) postoperative Schmerzbehandlung : Mit kontrollierter Patienten-Eigensteuerung und Verringerung ;

c) Schmerzbehandlung in der Geburtshilfe : Im Prinzip wie unter b), und

d) Intensivmedizin : Kontrollierte Infusion von z. B. Strophanthin beim Herzinfarkt mit automatischer Verringerung über 24 Stunden.

Einige Beispiele zu den Einsatzgebieten sollen nachfolgend angegeben werden.

## Beispiel 1

Intraoperativ : 0,2 % Tramadol-Lösung, Minimalgeschwindigkeit (Minimaldosis) als konstante Geschwindigkeit von 12 Tropfen/min., keine refraktäre Pause, Maximalgeschwindigkeit (Maximaldosis) 99 Tropfen/min., Verringerung in dem I. Intervall (I. Verringerungsphase) 20 Tropfen/min., in der II. Verringerungsphase 9 Tropfen/min., Beginn der Verringerung nach 15 Minuten und Dauer der einzelnen Stufen in dem I. und II. Intervall bzw. in der I. und II. Verringerungsphase 5 Minuten.

Das bedeutet, daß die Dauer der Verringerungsphasen I. und II. zusammen 30 Minuten beträgt und daß danach mit Beginn der 45. Minute die Infusionsgeschwindigkeit konstant bis Operationsende 12 Tropfen/min. beträgt.

## Beispiel 2

Postoperative Schmerzbehandlung : 0,2 % Tramadol, Minimalgeschwindigkeit 6 Tropfen/min., refraktäre Pause 5 Minuten, Maximalgeschwindigkeit 99 Tropfen/min. über 2 Minuten (Infusionsimpulsdauer), Verringerung um 20 Tropfen/min., beginnend nach 60 Minuten, Dauer jeder einzelnen Verringerungsphase (= Intervall) ebenfalls 60 Minuten. Dabei wird die konstante Minimalgeschwindigkeit von 6 Tropfen/min. am Ende der 5. Stunde erreicht, entsprechend 36 mg Tramadol pro Stunde.

## Beispiel 3

Schmerzbehandlung in der Geburtshilfe : Im Prinzip ähnlich wie Beispiel 2.

## Beispiel 4

Intensivmedizin : Ein Patient mit frischem Herzinfarkt benötigt in kürzest möglicher Zeit die sogenannte Sättigungsdosis (z. B. von Strophanthin) und daran anschließend die Konstanthaltung als sogenannte Erhaltungsdosis. Ein derartiges Programm könnte etwa so aussehen : 1 mg Strophanthin in 500 ml, Minimalgeschwindigkeit 19 Tropfen/min., keine refraktäre Pause, Maximalgeschwindigkeit (Maximaldosis) 99 Tropfen/min., Maximaldosis über eine Laufzeit von 7 Tagen ; eine Verringerung von 5 Tropfen/min., beginnend nach 8 Stunden (480 Minuten), Dauer der einzelnen Verringerungsphase (= Intervall) 60 Minuten. Dann wird nach insgesamt 24 Stunden die konstante Minimalgeschwindigkeit als Erhaltungsdosis erreicht.

**Patentansprüche**

1. Gerät zur geregelten Infusion von Flüssigkeiten mit einem Flüssigkeitsvorratsbehälter und einer elektrisch angetriebenen Infusionspumpe, die die Flüssigkeit aus dem Vorratsbehälter in eine Infusionsleitung fördert und die nach einem austauschbaren oder veränderbaren Programm eines Programm- und Steuersignalgebers zur Erzielung bestimmter änderbarer Infusionsprofile angetrieben wird, wobei der Programm- und Steuersignalgeber (8)
entsprechend dem Programm der Infusionspumpe zuführbare Steuersignale zur stetigen oder stufenweisen Änderung der Infusionsgeschwindigkeit, der Infusionszeit und der zu infundierenden Gesamtmenge an Flüssigkeit in vorbestimmten zeitlichen Infusionsintervallen einer vorbestimmten Gesamtinfusionszeit erzeugt,
eine erste Schaltungsanordnung (28) zur Steuerung und Überwachung einer ersten Infusion mit vorgebbarer maximaler Strömungsgeschwindigkeit über eine vorgebbare Infusionszeit,
eine zweite Schaltungsanordnung (30, 32) zur Steuerung und Überwachung einer weiteren Infusion mit vorgebbarer stufenweiser oder stetiger Verringerung der Strömungsgeschwindigkeit der Flüssigkeit über ein vorgebbares Zeitintervall,
eine dritte Schaltungsanordnung (34) zur Steuerung und Überwachung der Länge der einzelnen Infusionsintervalle und zur Umschaltung auf den aufeinanderfolgenden Infusionsintervallen zugeordnete Vorgabewerte für die Strömungsgeschwindigkeit, für die in Stufen oder stetig zu erniedrigende, zu infundierende Flüssigkeitsmenge und für die Stufenzahl oder Infusionszeit,
dadurch gekennzeichnet, daß der Programm- und Steuergeber (8) eine vierte Schaltungsanordnung (36) aufweist zur Steuerung und Überwachung der jeweiligen Strömungsgeschwindigkeit der von der Infusionspumpe (22, 24) abgegebenen Flüssigkeit und zur Umschaltung der Infusionspumpe auf eine Basis-Strömungsgeschwindigkeit, wenn die stufenweise oder stetig verringerte aktuelle Strömungsgeschwindigkeit kleiner oder gleich dieser Basisgeschwindigkeit ist.

2. Gerät nach Anspruch 1, dadurch gekennzeichnet, daß der Programm- und Steuersignalgeber (8) eine fünfte für einen Bedarfsbetriebszustand zuschaltbare Schaltungsanordnung (38) aufweist zur Steuerung und Überwachung nach Bedarf manuell zusätzlich einsteuerbarer vorbestimmbarer Flüssigkeitsmengen, z. B. der Infusionsimpulse, in vorgebbarer Zeit in Abhängigkeit von der jeweiligen maximalen Infusionsmenge und der bereits infundierten Menge und zur Umschaltung der Infusionspumpe auf die Basis-Strömungsgeschwindigkeit nach Infusion dieser Flüssigkeitsmenge.

3. Gerät nach Anspruch 2, dadurch gekennzeichnet, daß die Schaltungsanordnung (38) ein Steuersignal zur Sperrung (90) weiterer Bedarfsinfusionen, z. B. der Infusionsimpulse, erzeugt, das erst nach Ablauf eines Zeitgliedes (86, 88), durch das eine sogenannte refraktäre Pause definiert wird, wieder zurückgesetzt wird.

4. Gerät nach Anspruch 1, dadurch gekennzeichnet, daß die Schaltungsanordnung (28) einen Speicher (44) aufweist, in dem ein einer bei maximaler Strömungsgeschwindigkeit zu infundierenden maximalen Flüssigkeitsanfangsmenge (Plateau 42) entsprechender Sollwert abgespeichert ist, welcher mit dem in einer Meßeinrichtung (46) laufend gemessenen Istwert der infundierten Flüssigkeitsmenge in einem Vergleicher (48) verglichen wird, welcher bei Erreichen des Sollwertes durch den Istwert ein Ausgangssignal abgibt, das als Steuersignal der zweiten Schaltungsanordnung (30, 32) zugeführt wird.

5. Gerät nach Anspruch 1 oder 4, dadurch gekennzeichnet, daß die zweite Schaltungsanordnung (30, 32) einen ersten Speicher (52) aufweist, in dem ein oder mehrere in dem Plateau (42) folgenden Infusionsintervallen (I ... IV) zu infundierende Flüssigkeitsmengen entsprechende, abrufbare Vorgabe-Sollwerte abgespeichert sind, die mit dem in einer Meßeinrichtung (54) laufend gemessenen Istwert der infundierten Flüssigkeitsmenge in einem Vergleicher (56) verglichen wird, welcher bei gleichem Ist- und Sollwert ein Ausgangssignal erzeugt, das einen Subtrahierer (58) ansteuert, dessen einer Eingang zur Übernahme des Vorgabe-Sollwertes mit dem ersten Speicher (52) und dessen anderer Eingang zur Übernahme eines Verringerungswertes mit einem zweiten Speicher (60) verbunden ist und an dessen Ausgang ein Signal erscheint, das dem sich aus Vorgabe-Sollwert und Verringerungswert ergebenden Differenzwert entspricht, welcher in den ersten Speicher (52) geladen wird, und das der dritten Schaltungsanordnung (34) zugeführt wird.

6. Gerät nach Anspruch 5, dadurch gekennzeichnet, daß der Differenzwert vor der Eingabe in den ersten Speicher (52) in einem Zwischenspeicher (62) zwischengespeichert wird.

7. Gerät nach Anspruch 5, dadurch gekennzeichnet, daß das Ausgangssignal des Subtrahierers (58) aufeinanderfolgend die im Speicher (52) gespeicherten Vorgabe-Sollwerte abruft.

8. Gerät nach Anspruch 1, 5 oder 6, dadurch gekennzeichnet, daß die dritte Schaltungsanordnung (34) einen Speicher (66) aufweist, in dem ein oder mehrere der Zahl von Subtraktionsvorgängen bzw. der zugeordneten Stufenzahl entsprechende Sollwerte gespeichert sind, welche mit dem die Subtraktionsvorgänge bzw. Ausgangssignale des Subtrahierers (58) oder Zwischenspeichers (62) anzeigenden Ist-Zählerstand eines Zählers (64) in einem Vergleicher (68) verglichen werden, an dessen Ausgang bei Gleichstand von Ist- und Sollwert ein Ausgangssignal ansteht.

9. Gerät nach Anspruch 8, dadurch gekennzeichnet, daß das Ausgangssignal des Vergleichers (68) für das Folgeinfusionsintervall (I, II, III oder IV) die Übertragung eines neuen Vorgabe-Sollwertes aus einem Speicher (70) in den Speicher (52) steuert.

10. Gerät nach Anspruch 8, dadurch gekennzeichnet, daß das Ausgangssignal des Vergleichers (68) für das Folgeinfusionsintervall die Aktivierung oder Übertragung eines neuen Verringerungswertes im oder in den Speicher (66) steuert.

11. Gerät nach Anspruch 1, dadurch gekennzeichnet, daß die vierte Schaltungsanordnung (36) einen Speicher (50) aufweist, in dem ein der jeweils aktuellen Strömungsgeschwindigkeit entsprechender Signalwert für die Steuerung der Infusionspumpe abgespeichert ist, ferner einen Subtrahierer (74), der vom Ausgangssignal des Vergleichers (56) der zweiten Schaltungsanordnung (30, 32) angesteuert wird, an dessen einem Eingang der Signalwert eines Speichers (76) abgespeichert ist, in dem ein der jeweils höchsten Strömungsgeschwindigkeit entsprechender Signalwert und an dessen anderem Eingang der Ausgang eines Speichers (72) angeschlossen ist, in dem ein oder mehrere Verringerungswerte abgespeichert sind, und an dessen Ausgang ein Signal erscheint, das sich aus dem Wert des Speichers (76) und dem Verringerungswert des Speichers (72) ergebenden Differenzwert entspricht, welcher mit dem in einem Speicher (80) abgespeicherten Basis-Strömungsgeschwindigkeitswert in einem Vergleicher (78) verglichen wird, dessen Ausgangssignal die Übertragung des Differenzwertes als neuen aktuellen Strömungsgeschwindigkeitswert in den Speicher (50) steuert, wenn der Differenzwert den Basis-Strömungsgeschwindigkeitswert überschreitet und dessen Ausgangssignal die Übertragung des Basis-Strömungsgeschwindigkeitswertes in den Speicher (50) steuert, wenn der Differenzwert gleich dem Basis-Strömungsgeschwindigkeitswert ist oder diesen unterschreitet.

12. Gerät nach Anspruch 2 oder 3, dadurch gekennzeichnet, daß die fünfte Schaltungsanordnung (38) einen Bedarfsschalter (21) aufweist, durch dessen bei Betätigung abgegebenes Ausgangssignal über eine in den Durchlaßzustand schaltbare Sperreinrichtung (90) eine Einrichtung zur Erhöhung der Flüssigkeits-Strömungsgeschwindigkeit durch die Infusionspumpe (22) ansteuerbar ist für einen zusätzlichen Infusionsimpuls.

13. Gerät nach Anspruch 12, dadurch gekennzeichnet, daß die Einrichtung einen Speicher (82) aufweist, in dem vorgebbare Bedarfsmengenwerte abgespeichert sind.

14. Gerät nach Anspruch 13, dadurch gekennzeichnet, daß dem Speicher (82) ein Zähler (84) nachgeschaltet ist, dessen Zählerstand auf den jeweils aktuellen Inhalt des Speichers (82) setzbar ist und der bei Ansteuerung des Zählers heruntergezählt wird und der ferner bei Erreichung des Zählerstandes 0 ein Ausgangssignal erzeugt, das die Übertragung des Inhalts des Speichers (80) in den Speicher (50) bewirkt und gleichzeitig einen Speicher (86) ansteuert, in dem vorgebbare Pausewerte für eine nachfolgende refraktäre Pause abgespeichert sind und dem ein Ablaufzähler (88) nachgeschaltet ist, dessen Zählerstand auf den Inhalt des Speichers (86) setzbar und herunterzählbar ist und der bei Erreichen des Zählerstandes 0 ein Steuersignal zur Beendigung der refraktären Pause abgibt.

15. Gerät nach Anspruch 12 oder 14 und einem der vorhergehenden Ansprüche, dadurch gekennzeichnet, daß die Sperreinrichtung (90) durch Schließen eines Programmschalters (20) zur Umschaltung zwischen Automatik- und Bedarfsbetrieb durch das Ausgangssignal des Vergleichers (48) der ersten Schaltungsanordnung (28) in den leitenden Zustand und durch das dem Speicher (82) zugeführte wirksame Bedarfssignal des Bedarfsschalters (21) wieder in den nichtleitenden Zustand zurückgeschaltet und im Automatikbetriebszustand des Infusionsgerätes, d. h. bei offenem Schalter (20), in einen Dauer-Sperrzustand geschaltet ist.

16. Gerät nach einem der Ansprüche 12 bis 15, dadurch gekennzeichnet, daß die Bedarfsschalter-Signale des Bedarfsschalters (21) und die von der Sperreinrichtung (90) durchgelassenen wirksamen Bedarfsschaltersignale in zugeordneten Zählern (92 und 94) gezählt werden, deren Zählerstände durch einen Vergleicher (96) laufend miteinander verglichen werden, welcher bei Überschreitung einer vorgebbaren Differenz zwischen beiden Zählerständen ein Ausgangssignal zur Ansteuerung einer Warneinrichtung erzeugt.

17. Gerät nach Anspruch 11 oder einem der vorhergehenden Ansprüche, dadurch gekennzeichnet, daß die Schaltungsanordnung (36) eine Steuereinrichtung (101) aufweist, über die ein erster, normalerweise offener, zwischen dem Basis-Geschwindigkeitswert-Speicher (80) und dem Speicher (50) für den aktuellen Strömungsgeschwindigkeitswert angeordneter Schalter (103) und ein zweiter, normalerweise geschlossener, zwischen dem Speicher (76) für die jeweils laufende höchste Strömungsgeschwindigkeit und dem Speicher (50) angeordneter Schalter (102) umschaltbar sind in Abhängigkeit vom Ausgangssignal des Vergleichers (48) der Schaltungsanordnung (28), des Vergleichers (78) der Schaltungsanordnung (36), des wirksamen Bedarfsschaltersignals des Bedarfsschalters (21) und des Zählers (84) der Schaltungsanordnung (38), wobei das Ausgangssignal des Vergleichers (78) die Umschaltung des Schalters (103) in den Schließzustand und des Schalters (102) in den Offenzustand bewirkt, wenn der Differenzwert gleich dem Basis-Strömungsgeschwindigkeitswert ist oder diesen unterschreitet, zur Übertragung des Basis-Strömungsgeschwindigkeitswertes in den Speicher (50), und die Umschaltung des Schalters (103) in die Offenstellung und des Schalters (102) in die Schließstellung, wenn der Differenzwert größer ist als der Basis-Strömungsgeschwindigkeitswert, zur Übertragung des Differenzwertes in den Speicher (50).

18. Gerät nach Anspruch 17 und 15, dadurch gekennzeichnet, daß das Ausgangssignal des Vergleichers (48) gleichzeitig die Steuereinrichtung (101) betätigt zur Umschaltung des Schalters (103) in den Schließzustand und des Schalters (102) in den Offenzustand zur Übertragung des Inhalts des Speichers (80) in den Speicher (50).

19. Gerät nach Anspruch 17 und 15, dadurch gekennzeichnet, daß das wirksame Bedarfssignal des Bedarfsschalters (21) ebenfalls der Steuereinrichtung (101) zugeführt wird zur Umschaltung des Schalters (103) in den Offenzustand und des Schalters (102) in den Schließzustand zur Übertragung des Inhalts des Speichers (76) in den Speicher (50).

20. Gerät nach Anspruch 17 und 14, dadurch gekennzeichnet, daß zur Übertragung des Inhalts des Speichers (80) in den Speicher (50) nach Ablauf des Zählers (84) das Ausgangssignal des Zählers (84) der Steuereinrichtung (101) zugeführt wird zur Umschaltung des Schalters (103) in den Schließzustand und des Schalters (102) in den Offenzustand.

21. Gerät nach Anspruch 14, dadurch gekennzeichnet, daß das Steuersignal des Ablaufzählers (88) die Sperreinrichtung (90) in den leitenden Zustand schaltet.

22. Gerät nach einem der vorhergehenden Ansprüche, dadurch gekennzeichnet, daß ein einstellbarer Ablaufzeitgeber (104) vorgesehen ist, der mit Programmbeginn, z. B. bei Automatik- oder Bedarfsbetrieb, gestartet wird und der nach Ablauf ein Steuersignal erzeugt, das den Speicher (50) löscht oder dessen Inhalt auf den Strömungsgeschwindigkeitswert Null setzt.

23. Gerät nach einem der vorhergehenden Ansprüche, dadurch gekennzeichnet, daß nach Ablauf des Bedarfsbetriebs der im Speicher (80) abgespeicherete Basis-Strömungsgeschwindigkeitswert über den Vergleicher (78) in den Speicher (76) für den jeweils höchsten Strömungsgeschwindigkeitswert abgespeichert wird.

24. Gerät nach Anspruch 1, dadurch gekennzeichnet, daß der Programm- und Steuersignalgeber ein programmierbarer Prozessor (102) ist, der nach einem in einem Programmspeicher (104) gespeicherten, über eine Eingabeeinheit (106) änderbaren Programm die Infusionspumpe (22) steuert und die Infusionsvorgänge überwacht, und daß eine Ausgabeeinheit (110) zur optischen und/oder grafischen Wiedergabe der Eingabedaten, des Infusionsprofils und der laufenden Infusionsdaten vorgesehen ist.

**Claims**

1. Apparatus for the controlled perfusion of liquids with a reservoir for the liquid and an electrically driven perfusion pump, which transports the liquid from the reservoir and is driven in accordance with a replaceable or modifiable program from a program and control signal generator to give specific modifiable perfusion profiles, where the program and control signal generator (8)

generates control signals in accordance with the program to be sent to the perfusion pump for the constant or phased modification of the rate of perfusion, the period of perfusion and the total quantity of liquid to be perfused at predetermined perfusion intervals within a predetermined total period of perfusion,

has a first circuit configuration (28) for controlling and monitoring a first perfusion with presettable maximum flow rate over a presettable perfusion period,

has a second circuit configuration (30, 32) for controlling and monitoring a further perfusion with a presettable staged or constant reduction of the liquid flow rate over a presettable time interval,

has a third circuit configuration (34) for controlling and monitoring the length of the individual perfusion intervals and for switching to the preset values for the flow rate, for the quantity of perfusion liquid to be reduced in stages or constantly and for the number of stages or perfusion period allocated to the subsequent perfusion intervals,

characterized by the fact that the program and control signal generator (8) has a fourth circuit configuration for controlling and monitoring the flow rate of the liquid supplied by the perfusion pump (22, 24) at any time and for switching the perfusion pump to a base flow rate if the current flow rate, having been reduced in stages or constantly, is less than or equal to this base rate.

2. Apparatus as described in claim 1, characterized by the fact that the program and control signal generator (8) has a fifth circuit configuration (38) selectable for a demand operating mode for controlling and monitoring additional manually predetermined liquid quantities, e.g. a perfusion pulse, in a preset time dependent upon the maximum perfusion quantity at any time and the quantity already supplied and to switch the perfusion pump to the base flow rate once this quantity of liquid has been supplied.

3. Apparatus as described in claim 1, characterized by the fact that circuit configuration (38) generates a control signal to shut off (90) further demand perfusion, e. g. the perfusion pulse, which is only reset again after a time function element (86, 88), by which means a « refractory » pause is defined, has elapsed.

4. Apparatus as described in claim 1, characterized by the fact that the circuit configuration (28) includes a memory (44) in which a specified value corresponding to a maximum initial quantity of liquid to be perfused (plateau 42) at maximum flow rate is stored, which value is compared with the actual value for the quantity of liquid perfused, as continually metered in a measuring device (46), in a comparator circuit (48), which feeds an output signal into the second circuit configuration (30, 32) as a control signal when the actual value reaches the specified value.

5. Apparatus as described in claim 1 or 4, characterized by the fact that the second circuit configuration (30, 32) includes a first memory (52), in which one or more setpoint values corresponding to the quantities of liquid to be perfused at the perfusion intervals (I ... IV) following plateau (42) are stored, which values are compared with the actual value for the quantity of liquid perfused, as continually metered in a measuring device (54), in a comparator circuit (56), which, when the actual value reaches the specified value, issues an output signal controlling a subtractor, the one input of which is connected to the first memory (52) to receive the specified value and the second input of which is connected to a second memory (60) to receive a reduction value and the output of which issues a signal corresponding to the difference value of the setpoint value and the reduction value, which is then loaded into the first memory (52) and fed to the third circuit configuration (34).

6. Apparatus as described in claim 5 characterized by the fact that the difference value is stored temporarily in an intermediate memory (62) before input into the first memory (52).

7. Apparatus as described in claim 5 characterized by the fact that the output signal from the subtractor (58) calls up the setpoint values stored in memory (52) in sequence.

8. Apparatus as described in claims 1, 5 or 6, characterized by the fact that the third circuit configuration (34) includes a memory (66) in which one or more of the specified values corresponding to the number of subtraction procedures or the allocated number of stages are stored, which values are compared with the actual reading of a counter (64) indicating the subtraction procedures or output signals from the subtractor (58) or intermediate memory (62) in a comparator (68), which generates an output signal at its output when the specified and actual values are equal.

9. Apparatus as described in claim 8, characterized by the fact that the output signal from the comparator (68) controls the transfer of a new setpoint value from a memory (70) into memory (52) for the next perfusion interval (I, II, III or IV).

10. Apparatus as described in claim 8, characterized by the fact that the output signal from comparator (68) controls the activation or transfer of a new reduction value into the memory or memories (66) for the next perfusion interval.

11. Apparatus as described in claim 1 characterized by the fact that the fourth circuit configuration (36) includes a memory (50) in which a signal value for the control of the perfusion pump corresponding to the flow rate current at any time is stored, and in addition, a subtractor circuit (74), driven by the output signal of the comparator (56) of the second circuit configuration (30, 32), the one input of which stores the signal value of a memory (76) in which a signal value corresponding to the maximum flow rate current at the time and the other input of which is connected to the output from a memory (72) in which one or more reduction values are stored and the output of which issues a signal corresponding to the differential between the value in memory (76) and the reduction value in memory (72), this difference value being compared with the base flow rate value stored in a memory (80) in a comparator (78), the output signal of which controls the transfer of the difference value into memory (50) as a new current flow rate, if the difference value exceeds the base flow rate value and its output signal controls the transfer of the base flow rate value into memory (50), if the difference value is equal to or less than the base flow rate value.

12. Apparatus as described in claim 2 or 3, characterized by the fact that the fifth circuit configuration (38) includes a demand switch (21) and the output signal issued when this switch is operated can be used to drive a device for increasing the liquid flow rate through the perfusion pump (22) for an additional perfusion pulse via a shut-off device (90) switchable to the on state.

13. Apparatus as described in claim 12 characterized by the fact that the device includes a memory (82) in which presettable values for demand quantities are stored.

14. Apparatus as described in claim 13, characterized by the fact that a counter (84) is connected after memory (82), the counter reading of which may be set to the current content of the memory (82) at any time and counts down when the counter is driven and which also generates an output signal when the counter reading reaches zero causing the content of memory (80) to be transferred to memory (50) and simultaneously driving a memory (86), in which presettable pause values for a subsequent refractory pause are stored and to which an operation counter (88) is connected, the counter indication of which can be set to the content of memory (86) and can be counted down and which issues a control signal to end the refractory pause when the counter indication reaches zero.

15. Apparatus as described in claim 12 or 14 and one of the previous claims characterized by the fact that, if the program switch (20) is closed, the shut-off device (90) may be switched between automatic and demand operation with the output signal of a comparator (48) of the first circuit configuration (28) switching it into the on-mode and the effective demand signal from demand switch (21) sent to memory (82) switching it into the off-mode and, if the perfusion device is in automatic demand operation, i. e. if program switch (20) is open, the device is switched into a permanent shut-off state.

16. Apparatus as described in one of the claims 12 to 15, characterized by the fact that the demand switch signals from the demand switch (21) and the active demand switch signals allowed to pass by the shut-off device (90) are counted in allocated counters (92 and 94), the counter states of which are continuously compared to one another by a comparator (96), which generates an output signal to drive a warning device if a preset differential between the two counter states is exceeded.

17. Apparatus as described in claim 11 or one of the previous claims characterized by the fact that the circuit configuration (36) includes a control device (101), via which a first, normally open, switch (103) mounted between the base rate memory (80) and the memory (50) for the currently valid flow rate and a second, normally closed, switch (102) connected between the memory (76) for the maximum flow rate effective at any time and memory (50) are switchable depending upon the output signal from the comparator (48) of circuit configuration (28), the comparator of circuit configuration (36), the active demand switch signal from demand switch (21) and the counter (84) of circuit configuration (38), with the output signal from the comparator (78) switching switch (103) into the closed state and switch (102) into the open state if the difference value is equal to the base flow rate value or is less than.this, to transfer the base flow rate value into memory (50), and switching switch (103) into the open position and switch (102) into the closed position if the difference value is greater than the base flow rate value, to transfer the difference value into memory (50).

18. Apparatus as described in claims 17 and 15, characterized by the fact that the output signal from comparator (48) simultaneously triggers control device (101) to switch switch (103) into the closed state and switch (102) into the open state to transfer the content of memory (80) into memory (50).

19. Apparatus as described in claims 17 and 15, characterized by the fact that the active demand signal from demand switch (21) is also sent to control device (101) to switch switch (103) into the open state and switch (102) into the closed state, to transfer the content of memory (76) into memory (50).

20. Apparatus as described in claims 17 and 14, characterized by the fact that the output signal from counter (84) is sent to control device (101) once the counter has counted down to zero to transfer the content of memory (80) into memory (50) in order to switch switch (103) into the closed state and switch (102) into the open state.

21. Apparatus as described in claim 14 characterized by the fact that the control signal from the operation counter (88) switches the shut-off device (90) into the on condition.

22. Apparatus as described in one of the above claims, characterized by the fact that a settable elapsed time signal generator (104) is provided which is started as the program begins, e. g. in automatic or demand operation, and which, when it has counted down, generates a control signal to erase memory (50) or set its content to a flow rate value of zero.

23. Apparatus as described in one of the above claims, characterized by the fact that, after demand operation is completed, the base flow rate value stored in memory (80) is stored by comparator (78) in memory (76) for the maximum flow rate value valid at any time.

24. Apparatus as described in claim 1, characterized by the fact that the program and control signal generator is a programmable processor (102) which processes in accordance with a program which is stored in a program memory (104) and is modifiable via an input device (106) and that an output device (110) is provided for optical and/or graphic display of the input data, the perfusion profiles and the current perfusion data.

## Revendications

1. Appareil pour l'infusion réglée de liquides avec un réservoir de stockage de liquide et une pompe d'infusion actionnée électriquement, qui refoule le liquide du réservoir de stockage dans une conduite d'infusion et qui est actionnée avec un programme échangeable ou modifiable d'un émetteur de programme et de signaux de commande pour l'obtention d'un profilé d'infusion déterminé modifiable, l'émetteur de programme et les signaux de commande (8) produisant des signaux de commande pouvant être envoyés à la pompe d'infusion en correspondance avec le programme pour la modification continue ou graduelle de la vitesse d'infusion, du temps d'infusion et de la quantité totale de fluide à infuser dans des intervalles d'infusion temporaires prédéterminés d'un temps d'infusion total prédéterminé,

une première structure de câblage (28) pour la commande et la surveillance d'une première infusion avec une vitesse d'écoulement maximale prédéterminable pendant un temps d'infusion prédéterminable,

une deuxième structure de câblage (30, 32) pour la commande et la surveillance d'une infusion supplémentaire avec diminution prédéterminable étagée ou continue de la vitesse d'écoulement du liquide pendant un intervalle de temps prédéterminable,

une troisième structure de câblage (34) pour la commande et la surveillance de la longueur des intervalles d'infusion individuels et pour la commutation sur les valeurs allouées attribuées à la vitesse d'écoulement sur les intervalles d'infusion se suivant les uns les autres, pour les quantités des liquides à infuser à diminuer par étages ou en continu et pour le nombre d'étages ou le temps d'infusion,

caractérisé en ce que l'émetteur de programme et de commande (8) présente une quatrième structure de câblage (36) pour la commande et la surveillance de la vitesse d'écoulement suivant le cas du liquide envoyé par la pompe d'infusion (22, 24) et pour la commutation de la pompe d'infusion à une vitesse d'écoulement de base lorsque la vitesse d'écoulement effective diminuée par étages ou en continu est inférieure ou égale à cette vitesse de base.

2. Appareil selon la revendication 1, caractérisé en ce que l'émetteur de programme et de signaux de commande (8) présente une cinquième structure de câblage (38) pouvant être commutée à un état de fonctionnement de besoin pour la commande et la surveillance selon les besoins de quantité de liquide prédéterminable pouvant être injectée manuellement en supplément, par exemple de l'impulsion d'infusion, dans un temps prédéterminable en dépendance de la quantité d'infusion maximale suivant le cas et de la quantité déjà infusée et pour la commutation de la pompe d'infusion à la vitesse d'écoulement de base après infusion de cette quantité de liquide.

3. Appareil selon la revendication 2, caractérisé en ce que la structure de câblage (38) émet un signal de commande pour le blocage (90) d'autres infusions de besoin, par exemple des impulsions d'infusion, qui n'est rétabli qu'après écoulement d'un organe de temps (86, 88), par lequel est défini ce qu'il est convenu d'appeler une pause réfractaire.

4. Appareil selon la revendication 1, caractérisé en ce que la structure de câblage (28) présente une mémoire (44) dans laquelle est mise en mémoire une valeur prescrite correspondant à une quantité de début de fluide (plateau 42) maximale à infuser pour une vitesse d'écoulement maximale, qui est comparée dans un comparateur (48) avec la valeur effective de la quantité de liquide infusé mesurée dans un dispositif de mesure (46), qui émet un signal de sortie lorsque la valeur effective atteint la valeur prescrite, qui est envoyé à la deuxième structure de câblage (30, 32) en tant que signal de commande.

5. Appareil selon la revendication 1 ou 4, caractérisé en ce que la deuxième structure de câblage (30, 32) présente une première mémoire (52) dans laquelle sont mises en mémoires une ou plusieurs valeurs prescrites allouées pouvant être appelées sélectivement correspondant à des quantités de liquide à infuser dans le plateau (42) aux intervalles d'infusion successivement (I ... IV), qui sont comparées dans un comparateur (56) avec la valeur effective de la quantité de liquide infusé mesurée en continu dans un dispositif de mesure (54) qui émet un signal de sortie pour des valeurs effective et prescrite égales, qui commande un soustracteur (58) dont une première entrée est reliée à la première mémoire (52) pour recevoir la valeur prescrite allouée et dont l'autre entrée est reliée à une deuxième mémoire (60) pour recevoir une valeur de diminution, et à la sortie duquel apparaît un signal qui correspond à la valeur de la différence existant entre la valeur prescrite allouée et la valeur de diminution, qui est chargé dans la première mémoire (52) et qui est envoyé à la troisième structure de câblage (34).

6. Appareil selon la revendication 5, caractérisé en ce que la valeur des différences est mise en mémoire intermédiaire dans une mémoire intermédiaire (62) avant l'envoi à la première mémoire (52).

7. Appareil selon la revendication 5, caractérisé en ce que le signal de sortie du soustracteur (58) appelle sélectivement à la suite les unes des autres les valeurs prescrites allouées mises en mémoire dans la mémoire (52).

8. Appareil selon la revendication 1, 5 ou 6, caractérisé en ce que la troisième structure de câblage (34) présente une mémoire (66) dans laquelle sont mises en mémoire une ou plusieurs valeurs prescrites correspondant au nombre des opérations de soustraction ou au nombre d'étages adjoint, qui sont comparées dans un comparateur (68) avec la position de comptage effective d'un compteur (64) indiquant les opérations de soustraction ou les signaux de sortie du soustracteur (58) ou de la mémoire intermédiaire (62), à la sortie duquel est présent un signal de sortie pour une position identique des valeurs effective et prescrite.

9. Appareil selon la revendication 8, caractérisé en ce que le signal de sortie du comparateur (68) pour l'intervalle d'infusion successif (I, II, III ou IV) commande la transmission d'une nouvelle valeur prescrite allouée depuis une mémoire (70) dans la mémoire (52).

10. Appareil selon la revendication 8, caractérisé en ce que le signal de sortie du comparateur (68) pour l'intervalle d'infusion successif commande l'activation ou la transmission d'une nouvelle valeur de diminution dans la ou les mémoires (66).

11. Appareil selon la revendication 1, caractérisé en ce que la troisième structure de câblage (36) présente une mémoire (50) dans laquelle est mise en mémoire la valeur de signal correspondant à la vitesse d'écoulement effective suivant le cas pour la commande de la pompe d'infusion, en outre un soustracteur (74) qui est commandé par le signal de sortie du comparateur (56) de la deuxième structure de câblage (30, 32), à une entrée duquel est envoyée la valeur effective d'une mémoire (76) dans laquelle est raccordée une valeur effective correspondant à la vitesse d'écoulement la plus élevée suivant le cas et à l'autre entrée duquel est appliquée une sortie de la mémoire (72), dans lequel sont mises en mémoire une ou plusieurs valeurs de diminution, et à la sortie duquel apparaît un signal qui correspond à la valeur de différence résultant de la valeur de la mémoire (76) et de la valeur de diminution de la mémoire (72), qui est comparée dans un comparateur (78) avec la valeur de vitesse d'écoulement de base mise en mémoire dans une mémoire (80) dont le signal de sortie commande la transmission de la mémoire (50) de la valeur de différence en tant qu'une nouvelle valeur de vitesse d'écoulement effective, lorsque la valeur de différence dépasse la valeur de vitesse d'écoulement de base et dont le signal de sortie commande la transmission de la valeur de la vitesse d'écoulement de base dans la mémoire (50) lorsque la valeur de différence est égale à la valeur de vitesse d'écoulement de base ou lui est inférieure.

12. Appareil selon la revendication 2 ou 3, caractérisé en ce que la cinquième structure de câblage (38) présente un interrupteur de besoin (21) par lequel un dispositif pour augmenter la vitesse d'écoulement du liquide par la pompe d'infusion (22) peut être commandé par une impulsion d'une infusion supplémentaire par son signal de sortie envoyé lors de l'actionnement à travers un dispositif de blocage (90) pouvant être commuté à l'état passant.

13. Appareil selon la revendication 12, caractérisé en ce que le dispositif présente une mémoire (42) dans laquelle sont mises en mémoire des valeurs de quantités de besoins pouvant être allouées.

14. Appareil selon la revendication 13, caractérisé en ce qu'un compteur (84) est disposé après la mémoire (82), dont l'état de comptage peut être ajusté sur le contenu effectif suivant le cas de la mémoire (82) et qui, lors de l'excitation du compteur, est décompté et qui, en outre, lorsqu'on atteint l'état du compteur 0, émet un signal de sortie qui actionne la transmission du contenu de la mémoire (80) dans la mémoire (50) et commande simultanément une mémoire (86) dans laquelle sont mises en mémoire des valeurs de pause pouvant être allouées pour une pause réfractaire ultérieurement et après laquelle est disposé un compteur d'écoulement (88) dont l'état de comptage peut être réglé sur le contenu de la mémoire (86) et qui peut être décompté et qui, lorsqu'on atteint la valeur de comptage 0, émet un signal de commande pour l'achèvement de la pause réfractaire.

15. Appareil selon la revendication 12 ou 14 et l'une des revendications précédentes, caractérisé en ce que le dispositif de blocage (90) est mis en circuit par fermeture d'un interrupteur de programme (20) pour la commutation entre le fonctionnement automatique et de besoin par le signal de sortie du comparateur (48) de la première structure de câblage (28) à l'état conducteur et par le signal de besoin actif de l'interrupteur de besoin (21) envoyé à la mémoire (82) est recommuté à l'état de non-conduction et dans l'état de fonctionnement automatique de l'appareil d'infusion c'est-à-dire avec l'interrupteur (20) ouvert et commuté dans un état de blocage de durée.

16. Appareil selon l'une des revendications 12 à 15, caractérisé en ce que les signaux du compteur de besoin (21) et les signaux de compteur de besoin actifs ayant pu traverser le dispositif de blocage (90) sont comptés dans des compteurs (92 et 94) adjoints dont les états de comptage sont comparés entre eux en continu par un comparateur (96) qui, lors du dépassement d'une différence pouvant être allouée entre les deux valeurs de compteur, émet un signal de sortie pour la commande d'une installation d'alarme.

17. Appareil selon la revendication 11 ou l'une des revendications précédentes, caractérisé en ce que la structure de câblage (36) présente une installation de commande (101) par laquelle peuvent être commutés, en fonction du signal de sortie du comparateur (48) de la structure de câblage (28), du comparateur (78) de la structure de câblage (36), du signal d'interrupteur de besoin actif de l'interrupteur de besoin (21) et du compteur de l'installation de commutation, un premier interrupteur (103) normalement ouvert disposé entre la mémoire de valeur de vitesse de base (80) et la mémoire (50) pour la valeur de vitesse d'écoulement effective et un deuxième interrupteur (102) normalement fermé disposé entre la mémoire (76) pour la vitesse d'écoulement la plus élevée en continu suivant le cas et la mémoire (50), le signal de sortie du comparateur (78) actionnant la commutation de l'interrupteur (103) à l'état de fermeture et de l'interrupteur (102) à l'état d'ouverture lorsque la valeur de différence est égale à la valeur de la vitesse d'écoulement de base ou lui est inférieure, pour la transmission de la valeur de la vitesse d'écoulement de base dans la mémoire (50), et la commutation de l'interrupteur (103) en position d'ouverture et de l'interrupteur (102) en position de fermeture lorsque la valeur de différence est supérieure à la valeur de la vitesse d'écoulement de base pour la transmission de la valeur de différence dans la mémoire (50).

18. Appareil selon la revendication 17 ou 15, caractérisé en ce que le signal de sortie du comparateur (48) actionne simultanément le dispositif de commande (101) pour la commutation de l'interrupteur (103) à l'état de fermeture et de l'interrupteur (102) à l'état d'ouverture pour la transmission du contenu de la mémoire (80) dans la mémoire (50).

19. Appareil selon la revendication 17 ou 15, caractérisé en ce que le signal de besoin actif de l'interrupteur de besoin (21) est également envoyé au dispositif de commande (101) pour la commutation de l'interrupteur (103) à l'état d'ouverture et de l'interrupteur (102) à l'état de fermeture pour la transmission du contenu de la mémoire (76) dans la mémoire (50).

20. Appareil selon la revendication 17 ou 14, caractérisé en ce que, pour la transmission du contenu de la mémoire (80) dans la mémoire (50) après écoulement du compteur (84), le signal de sortie du compteur (84) est envoyé au dispositif de commande (101) pour la commutation de l'interrupteur (103) à l'état de fermeture et de l'interrupteur (102) à l'état d'ouverture.

21. Appareil selon la revendication 14, caractérisé en ce que le signal de commande du compteur d'écoulement (88) commande le dispositif de blocage (90) en l'état conducteur.

22. Appareil selon l'une des revendications précédentes, caractérisé en ce qu'un émetteur de temps d'écoulement (104) réglable est prévu, avec lequel est démarré le début de programme, par exemple en fonctionnement automatique ou de besoin, et qui, après écoulement, émet un signal de commande qui éteint la mémoire (50) et met à zéro son contenu de la valeur de vitesse d'écoulement.

23. Appareil selon l'une des revendications précédentes, caractérisé en ce que, après écoulement du fonctionnement de besoin, la valeur de vitesse d'écoulement de base mise en mémoire dans la mémoire (80) est mise en mémoire par l'intermédiaire du comparateur (78) dans la mémoire (76) pour la valeur de vitesse d'écoulement la plus élevée suivant le cas.

24. Appareil selon la revendication 1, caractérisé en ce que l'émetteur de programme et de signaux de commande est un processeur programmable (102) qui commande la pompe d'infusion (22) selon un programme modifiable par une unité d'entrée (106) mémorisée dans une mémoire de programme (104) et surveille les opérations d'infusion, et qu'une unité de sortie (110) est prévue pour la reproduction optique et/ou graphique des données d'entrée du profil d'infusion et de données d'infusion continue.

FIG. 1

FIG. 2

FIG. 3

FIG. 4

FIG. 5

EP 0 116 568 B1

# FIG. 6

100

Prozessor ~102

Interface

108

Programmspeicher ~104

Eingabeeinheit ~106

Ausgabeeinheit ~110

EP 0 116 568 B1